# EUROPEAN PATENT SPECIFICATION

(11) **EP 2 105 055 B1**
(45) Date of publication and mention of the grant of the patent: **22.01.2014**
(21) Application number: 09161674.8
(22) Date of filing: 06.10.2006
(51) Int. Cl.: A23L 1/29, A23L 1/30, A61K 31/202, A61K 31/7068, A61K 31/7072, A61K 31/7076, A61K 31/708, A61P 1/12, A23L 1/201

(54) **Method for stimulating intestinal barrier integrity after birth by caesarean section**
Verfahren zur Stimulation der Darmbarriere-Integrität nach Geburt durch Kayserschnitt
Procédé de stimulation de l'intégrité de la barrière intestinale après une naissance par césarienne

(30) Priority: 21.10.2005 EP 05023029
(43) Date of publication of application: 30.09.2009
(62) Divisional of application: 06799538.1
(73) Proprietor: N.V. Nutricia, 2700 MA Zoetermeer (NL)
(72) Inventor: Schmitt, Joachim, D-63768, Hoesbach (DE); Boehm, Günther, DE-61209, Echzell (DE); Beermann, Christopher, D-61267, Neu-Anspach (DE)
(74) Representative: Nederlandsch Octrooibureau

(56) References cited:
- EP-A- 1 634 599
- WO-A-2004/032651
- WO-A-2005/122790
- WO-A2-2004/112509
- US-A1- 2004 143 013
- HALLSTROM M, EEROLA E, VUENTO R, JANAS M, TAMMELA O: "Effects of the mode of delivery and necrotising enterocolitis on the intestinal microflora in preterm infants" EUR. J. CLIN. MICROBIOL. INFECT. DIS., vol. 23, 2004, pages 463-470, XP002420118
- FANARO S ET AL: "INTESTINAL MICROFLORA IN EARLY INFANCY: COMPOSITION AND DEVELOPMENT" ACTA PAEDIATRICA, UNIVERSITETSFORLAGET, OSLO, NO, no. SUPPL 441, September 2003 (2003-09), pages 48-55, XP009062406 ISSN: 0803-5253
- KOLETZKO B ET AL: "POLYUNSATURATED FATTY ACIDS IN HUMAN MILK AND THEIR ROLE IN EARLY INFANT DEVELOPMENT" JOURNAL OF MAMMARY GLAND BIOLOGY AND NEOPLASIA, PLENUM PRESS, NEW YORK, NY,, US, vol. 4, no. 3, July 1999 (1999-07), pages 269-284, XP009024376 ISSN: 1083-3021
- JANAS L M ET AL: "THE NUCLEOTIDE PROFILE OF HUMAN MILK" PEDIATRIC RESEARCH, WILLIAMS AND WILKINS, BALTIMORE, MD,, US, vol. 16, no. 8, August 1982 (1982-08), pages 659-662, XP002048454 ISSN: 0031-3998
- KUNZ C ET AL: "OLIGOSACCHARIDES IN HUMAN MILK: STRUCTURAL, FUNCTIONAL, AND METABOLIC ASPECTS" ANNUAL REVIEW OF NUTRITION, ANNUAL REVIEWS INC., PALO ALTO, CA, US, vol. 20, 2000, pages 699-722, XP009020860 ISSN: 0199-9885
- LAUBEREAU B ET AL: "Caesarean section and gastrointestinal symptoms, atopic dermatitis, and sensitisation during the first year of life", ARCH. DIS. CHILD., vol. 89, 2004, pages 993-997, DOI: 10.1136(adc.2003.043265
- HEKMATDOOST A ET AL: "The effect of dietary oils on cecal microflora in experimental colitis in mice", INDIAN J GASTROENTEROL, vol. 27, 2008, pages 186-189,

## Description

### FIELD OF THE INVENTION

The present invention relates to methods for feeding and therapy infants delivered via caesarean section.

### BACKGROUND OF THE INVENTION

Infants have an immature intestine at birth. After birth, the neonatal gastro-intestinal tract undergoes rapid growth and maturation, including mucus formation and maturation and closing of tight junctions to allergens, toxins and pathogenic micro-organisms. In human milk several factors are present which improve gut maturation.

A permeable, immature intestinal barrier plays a role in the acquirement of intestinal inflammation, infections, diarrhea, and atopic diseases including food allergy. A properly maturing intestine, especially in combination with a healthy intestinal flora, results in a reduced incidence of infections, a strengthened immune system, a reduced incidence of food allergy and/or other atopic diseases, and/or a reduced incidence of diarrhea, constipation and/or intestinal inflammation.

Intestinal permeability decreases faster in breast-fed babies than in formula-fed infants. However, not always is breast-feeding realizable.

W02004112509 describes a composition for inducing a pattern of intestinal barrier maturation similar to that observed with breast-feeding and able to improve intestinal barrier maturation, e.g. during neonatal stress.

WO2005122790 describes a method for stimulating barrier integrity in a mammal by administering to a mammal a composition comprising: eicosapentaenoic acid (EPA), docosahexaenoic acid (DHA) and arachidonic acid (ARA), and at least two distinct oligosaccharides.

EP1549158 describes infant formula compositions which comprise 3.2 mg/L to 15.4 mg/L of CMP; 1.8 mg/L to 11.0 mg/L of UMP; 1.8 mg/L to 8.0 mg/L of GMP; 0.1 mg/L to 2.2 mg/L of IMP; and 2.5 mg/L to 13.2 mg/L of AMP.

WO03043445 describes processed baby foods which are formulated with a) oils, b) nucleotides, c) oils and nucleotides.

Natren® produces the probiotic product Life Start® which is designed specifically for infants and suitable for infants delivered via caesarean section. Life Start® is made with *Bifidobacterium infantis*. Because the Life Start® product contains only one single *Bifidobacteria* species, the benefits for the infant will be very limited.

Lanbereau et al Arch. Dis. Child. 2004; 89 : 993-997 investigates the effect of caesarean section on gastrointestinal symptoms, atopic dermatitis and sensitization to nutritional allergens in infants.

### SUMMARY OF THE INVENTION

The present inventors have found that infants delivered via caesarean section have an intestinal flora which is different from the intestinal flora of infants born via the vaginal route. Particularly, infants born via caesarean section have a reduced rate of intestinal colonization by *Bifidobacteria* and have a less diverse *Bifidobacterium* intestinal flora regarding species than infants born via the vaginal route, particularly missing *Bifidobacterium breve, Bifidobacterium longum, Bifidobacterium infantis* and *Bifidobacterium bifidum*. It was further found that the intestinal flora of infants delivered via caesarean section have a lower content of *Bifidobacteria* compared to the intestinal flora of infants delivered vaginally. Additionally it was found that the intestinal flora of infants born via caesarean section has a high content of (undesirable) *Escherichia coli* 6 weeks after delivery.

A healthy and fully developed gastrointestinal flora has important physiological effects. One important aspect is that it reduces the occurrence of (gastrointestinal) infections. Because infants delivered via a caesarean section lack a healthy flora, preventing infection is particularly important for these infants. These infants are normally delivered in a hospital environment, which is a risk for pathogenic infection due to the occurrence of nosocomial bacteria. Additionally, the impaired development of a healthy intestinal flora results in faster colonization of pathogenic bacteria compared to a situation where the infants intestinal tract is inoculated by maternal bacteria originating from the mother's vaginal and fecal flora. In order to prevent bacterial translocation or translocation of bacterial (eno- or exo)toxins across the intestinal barrier of these (nosocomial) pathogens, it is of utmost importance to improve and/or accelerate intestinal maturation in caesarean section delivered infants.

Improvement of the intestinal barrier maturation and/or barrier integrity in caesarean section delivered infants furthermore reduces the incidence of intestinal inflammation, food allergy and/or other atopic diseases.

The inventors have recognized that the barrier function of babies born via caesarean section is impaired and found that long-chain polyunsaturated fatty acids (LC-PUFA) improve the maturation and integrity of the intestinal barrier, and/or have an anti-inflammatory effect in babies delivered via caesarean section. LC-PUFA furthermore promote the adhesion of lactic acid producing bacteria to mucosal surfaces. Hence, the present invention concerns the use of compositions comprising LC-PUFA that particularly aim to a) decrease the incidence of infections by increasing intestinal barrier integrity and/or maturation, b) decrease the incidence of (food) allergy and/or atopic diseases by increasing intestinal barrier integrity and/or maturation, c) dampen or subdue intestinal inflammatory processes that otherwise would further increase intestinal barrier permeability, and/or d) improve the flora by increasing colonization of lactic acid producing bacteria to mucosal surfaces in infants delivered by caesarean section.

In a further aspect the present invention can be suitably brought to practice by incorporation of the present active ingredients in a nutritional composition. Such composition can be administered to the infant without posing a heavy burden on the infant delivered via caesarean section.

### DETAILED DESCRIPTION OF THE PREFFERED EMBODIMENTS

The present invention provides the use of a composition comprising (i) long chain polyunsaturated fatty acid (LC-PUFA) for the manufacture of a composition for administration to an infant delivered via caesarean section as defined in claim 1.

### Caesarean section

A caesarean section (c-section) is a surgical procedure where an infant is delivered through an incision made in the mother's abdominal wall, and then through the wall of the uterus. A caesarean section is usually performed when it is safer for the mother or the infant than a vaginal delivery. Other times, a woman may choose to have a caesarean section rather than deliver her infant vaginally.

### Long-chain polyunsaturated fatty acids

Throughout this description, the composition as used in the method according to the present invention is often referred to as the "present composition". The present composition comprises long chain polyunsaturated fatty acids (LC-PUFA). LC-PUFA are fatty acids wherein the acyl chain has a length of 20 to 24 carbon atoms (preferably 20 or 22 carbon atoms) and wherein the acyl chain comprises at least two unsaturated bonds between said carbon atoms in the acyl chain. More preferably the present composition comprises at least one LC-PUFA selected from the group consisting of eicosapentaenoic acid (EPA, 20:5 n3), docosahexaenoic acid (DHA, 22:6 n3), arachidonic acid (ARA, 20:4 n6) and docosapentaenoic acid (DPA, 22:5 n3). EPA, DHA and ARA were found to effectively reduce intestinal tight junction permeability (see example 2). Reduced tight junction permeability reduces the occurrence of infection and/or reduces passage of allergens and/or reduces passage of bacterial (endo- or exo)toxins. Hence incorporation of LC-PUFA, preferably EPA, DHA, DPA and/or ARA, in the present composition improves intestinal barrier integrity, which is of utmost important for babies delivered via a caesarean section since these babies have a less developed intestinal flora and hence a slower maturing intestinal barrier. LC-PUFA further have anti-inflammatory effects and promote the adhesion of lactic acid producing bacteria to mucosal surfaces, thereby stimulating the development of a healthy flora, which are further advantages for use in caesarean section delivered infants.

Since a low concentration of ARA, DHA, DPA and/or EPA is already effective in reducing tight junction permeability, the content of LC-PUFA in the present composition, which is preferably a nutritional composition, preferably does not exceed 15 wt.% of the total fat content, preferably does not exceed 10 wt.%, even more preferably does not exceed 5 wt.%. Preferably the present composition comprises at least 0.1 wt.%, preferably at least 0.25 wt.%, more preferably at least 0.5 wt.%, even more preferably at least 0.75 wt.% LC-PUFA of the total fat content. For the same reason, the EPA content preferably does not exceed 5 wt.% of the total fat, more preferably does not exceed 1 wt.%, but is preferably at least 0.025 wt.%, more preferably at least 0.05 wt.% of the total fat. The DHA content preferably does not exceed 5 wt.%, more preferably does not exceed 1 wt.%, but is at least 0.1 wt.% of the total fat. As ARA was found to be particularly effective in reducing tight junction permeability, the present composition comprises relatively high amounts, preferably at least 0.1 wt.%, even more preferably at least 0.25 wt.%, most preferably at least 0.35 wt.% ARA of the total fat. The ARA content preferably does not exceed 5 wt.%, more preferably does not exceed 1 wt.% of the total fat. When the present enteral composition comprises ARA, EPA and DHA are advantageously added to balance the action of ARA, e.g. reduce the potential pro-inflammatory action of ARA metabolites. Excess metabolites from ARA may cause inflammation. Hence, the present composition preferably comprises ARA, EPA and DHA, wherein the weight ratio ARA/DHA preferably is above 0.25, preferably above 0.5, even more preferably above 1. The ratio ARA/DHA is preferably below 25, more preferably below 15. The weight ratio ARA/EPA is preferably between 1 and 100, more preferably between 5 and 20.

In order to mimic human breast milk as closely as possible and to reduce side effects of high dosages of LC-PUFA, in the present composition the content of LC-PUFA, preferably does not exceed 3 wt.% of the total fat content. The content of n3 (omega 3) LC-PUFA preferably is below 1 wt.% of the total fat content. The n6 (omega 6) LC-PUFA preferably is preferably below 2 wt.% of the total fat content. The ARA content is preferably below 1 wt.% of the total fat content. The weight ratio EPA/DHA preferably is 1 or below.

The LC-PUFA may be provided as free fatty acids, in triglyceride form, in diglyceride form, in monoglyceride form, in phospholipid form, or as a mixture of one of more of the above, preferably in triglyceride form. The present composition preferably comprises at least one of ARA and DHA in phospholipid form.

### Nucleotides

In a preferred embodiment the present composition further comprises nucleotide and/or nucleotide precursors selected from the group consisting of nucleoside, purine base, pyridine base, ribose and deoxyribose. More preferably the composition comprises nucleotide. The nucleotide is preferably in the monophosphate, diphosphate or triphosphate form, more preferably a nucleotide monophosphate. The nucleotide preferably is a ribonucleotide or a deoxyribonucleotide, more preferably a ribonucleotide. The nucleotides can be monomeric, dimeric or polymeric (including RNA and DNA). The nucleotides preferably are present as a free acid or in the form of a salt, more preferably monosodium salt. Incorporation of nucleotide in the present composition improves intestinal barrier integrity and/or maturation, which is of utmost importance for babies delivered via a caesarean section since these babies have a less developed intestinal flora and hence a slower maturing intestinal barrier. Preferably, the composition comprises one or more selected form the group consisting of cytidine 5'-monophospate (CMP), uridine 5'-monophospate (UMP), adenosine 5'-monophospate (AMP), guanosine 5'-monophospate (GMP), and inosine 5'-monophospate (IMP) and/or salts thereof, in particular monosodium salts thereof, more preferably at least 3 selected form the group consisting of CMP, UMP, AMP, GMP and IMP and/or salts thereof, in particular monosodium salts thereof. An increased diversity of nucleotides can further improve barrier integrity.

Preferably the composition comprises 5 to 100 mg, more preferably 5 to 50 mg, most preferably 10 to 25 mg nucleotides per 100 gram dry weight of the present composition. The nucleotides further stimulate the immune system thereby enhancing protection against a high load of intestinal pathogens such as *E. coli.*

Preferably the present composition comprises 1 to 20 mg, more preferably 3 to 12.5 mg CMP per 100 g dry weight of the composition. Preferably the composition comprises 1 to 20 mg, more preferably 2 to 9 mg UMP per 100 g dry weight of the composition. Preferably the present composition comprises 0.5 to 15 mg, more preferably 1.5 to 8 mg AMP per 100 g dry weight of the present composition. Preferably the composition comprises 0.2 to 10 mg, more preferably 0.6 to 3 mg GMP per 100 g dry weight of the composition. Preferably the present composition comprises 0.5 to 10 mg, more preferably 1.3 to 5 mg IMP per 100 g dry weight of the composition.

Preferably the present composition comprises both (i) LC-PUFA and (ii) nucleotide and/or nucleotide precursor selected from the group consisting of nucleosides, purine bases, pyridine bases, ribose and deoxyribose, since a combination of these components unexpectedly improve gut barrier function, immune system and/or intestinal flora, e.g. a synergistic effect is envisaged.

### Non-digestible oligosaccharides

The present composition preferably comprises non-digestible oligosaccharides which are fermented into organic acids (preferably lactic acid, butyrate, propionate and/or acetate) and stimulate the growth of the intestinal lactic acid producing bacteria (hereinafter referred to as "non-digestible saccharides"). Preferably the growth *Bifidobacteria* and/or *Lactobacilli* is stimulated. An increased content of *Bifidobacteria* and/or *Lactobacilli* stimulate the formation of a healthy intestinal flora. Additionally, the formed organic acids stimulate mucus production and therefore further decrease intestinal barrier permeability in infants delivered by caesarean section (see Example 3, below). The non-digestible oligosaccharides also enhance the effectiveness of the LC-PUFA in the present composition, as co-administration of the non-digestible oligosaccharides with the LC-PUFA causes a delay in absorption of the LC-PUFA in the small intestine, thereby prolonging and/or increasing the effects of the LC-PUFA in the colon. Advantageously, the present composition comprises non-digestible oligosaccharides preferably with a degree of polymerization (DP) of 2 to 250, more preferably 2 to 100, more preferably 2 to 10.

The present composition preferably contains non-digestible oligosaccharide with a degree of polymerization (DP) below 60, more preferably below 40, even more preferably below 20, most preferably below 10. Short chain non-digestible oligosaccharides provide an improved lactate and/or acetate production, stimulating the barrier integrity in the infant born via caesarean section. The non-digestible oligosaccharides are preferably not or only partially digested in the intestine by the action of acids or digestive enzymes present in the human upper digestive tract (small intestine and stomach) and are fermented by the human intestinal flora. For example, sucrose, lactose, maltose and the maltodextrins are considered digestible.

Preferably the present composition comprises at least one non-digestible oligosaccharide selected from the group consisting of fructo-oligosaccharides (including inulins), non-digestible dextrins, galacto-oligosaccharides (including transgalacto-oligosaccharides), xylo-oligosaccharides, arabino-oligosaccharides, arabinogalacto-oligosaccharides, gluco-oligosaccharides (including cyclodextrins and gentio-oligosaccharides), chito-oligosaccharides, glucomanno-oligosaccharides, galactomanno-oligosaccharides, mannan-oligosaccharides, fuco-oligosaccharides, galacturonic acid oligosaccharides, guluronic acid oligosaccharides, mannuronic acid oligosaccharides, iduronic acid oligosaccharides, riburonic acid oligosaccharides, glucuronic acid oligosaccharides and mixtures thereof, more preferably fructo-oligosaccharides, galacto-oligosaccharides (including transgalacto-oligosaccharides), fuco-oligosaccharides and galacturonic acid oligosaccharides and mixtures thereof, even more preferably galacto-oligosaccharides and/or inulin, most preferably transgalacto-oligosaccharides.

The present non-digestible oligosaccharide is preferably selected from the group consisting of galacto-oligosaccharides and fructo-oligosaccharides (e.g. inulin). Preferably at least 50 wt.% of the present non-digestible oligosaccharides have a degree of polymerization of 2 to 60. In a particular preferred embodiment the present composition comprises at least galacto-oligosaccharides and fructo-oligosaccharides. The galacto-oligosaccharides preferably comprise saccharides with a DP of 2 to 10. The fructo-oligosaccharides preferably comprise saccharides with a DP of 2 to 60.

Preferably, the galacto-oligosaccharide comprises beta bonds, as is the case in human milk oligosaccharides.

In a preferred embodiment the present composition comprises galacturonic acid oligosaccharides. The galacturonic acid oligosaccharides of the invention advantageously reduce the adhesion of pathogenic micro-organisms to the intestinal epithelial cells, thereby reducing colonization of (nosocomial) pathogenic bacteria in the colon of the infant delivered by caesarean section. Furthermore, the galacturonic acid oligosaccharides of the present invention stimulate the formation of a healthy intestinal flora and are fermented, resulting in a production of intestinal organic acids and a reduction of intestinal pH, which inhibit the growth of (nosocomial) pathogenic bacteria. The co-administration of galacturonic acid oligosaccharides therefore further improves protection for the infant delivered via caesarean section from infections due to the underdeveloped barrier function and/or underdeveloped intestinal bacterial flora.

The term galacturonic acid oligosaccharide as used in the present invention preferably refers to an oligosaccharide wherein at least 50% of the monosaccharide units present in the oligosaccharide are galacturonic acid. The present composition preferably comprises galacturonic acid oligosaccharide with a DP between 2 and 250, preferably 2 and 50, more preferably 2 and 20. The galacturonic acid oligosaccharides used in the invention are preferably prepared from pectin, pectate, and/or polygalacturonic acid. The galacturonic acid oligosaccharide is preferably derived from pectin. Preferably the pectin oligosaccharide is prepared by hydrolysis and/or beta-elimination of fruit pectin and/or vegetable pectin, more preferably from apple, citrus and/or sugar beet pectin, more preferably the apple, citrus and/or sugar beet pectin has been treated by at least a lyase. Preferably the pectin lysate and/or the galacturonic acid oligosaccharide is prepared from bacterial production.

In a preferred embodiment, at least one of the terminal hexuronic acid units of the galacturonic acid oligosaccharide has a double bond, which is preferably situated between the C₄ and C₅ position of the terminal hexuronic acid unit. The double bond effectively protects against attachment of pathogenic bacteria to intestinal epithelial cells. This is advantageous for infants delivered by caesarean section. Preferably at least 5%, more preferably at least 10%, even more preferably at least 25% of the terminal hexuronic acid units of the galacturonic acid oligosaccharide is an unsaturated hexuronic acid unit. As each individual galacturonic acid oligosaccharide preferably comprises only one unsaturated terminal hexuronic acid unit, preferably less than 50% of the terminal hexuronic acid units is an unsaturated hexuronic acid unit (i.e. comprises a double bond).

The present composition preferably comprises between 0.01 and 10 grams galacturonic acid oligosaccharide with a DP of 2 to 250 per 100 gram dry weight of the composition, more preferably between 0.05 and 6 gram, even more preferably 0.2 to 2 gram. Preferably the present composition comprises between 0.01 and 10 grams galacturonic acid oligosaccharide with a DP of 2 to 25 per 100 gram dry weight of the nutritional composition, more preferably between 0.05 and 6 gram, even more preferably 0.2 to 2 gram. The short chain galacturonic acid oligosaccharides are more effective and/or better suitable for inclusion in the present composition.

Preferably, the composition comprises (i) galacto-oligosaccharides, (ii) fructo-oligosaccarides and (iii) galacturonic acid oligosaccharides. This combination is particularly suitable for protection the infants delivered via caesarean section as it provides an optimal stimulation of the *Bifodobacteria* and/or *Lactobacilli* and reduces adherence of pathogenic organisms and reduces colonization by and/or adherence of potential pathogenic bacteria such as *E. coli.*

### Lactic acid producing bacteria

The present composition preferably comprises lactic acid producing bacteria, either living or dead. Dead bacteria can for example be prepared by inactivating living bacteria by heat treatment and/or sonication. Living lactic acid bacteria are advantageously incorporated to stimulate fast colonization of the infant's intestinal tract. Lactic acid producing bacteria are preferably provided as a mono- or mixed culture of live microorganisms. The present composition preferably comprises 10² to 10¹³ colony forming units (cfu) of lactic acid producing bacteria per gram dry weight of the present composition, preferably 10² to 10¹² cfu, more preferably 10⁵ to 10¹⁰ cfu, most preferably from 10⁴ to 5x10⁹ cfu.

Preferably the present composition comprises bacteria of the genus *Lactobacillus* and/or *Bifidobacterium.* Preferably the composition comprises at least one *Bifidobacterium* selected from the group consisting of *B. longum, B. breve, B. infantis, B. catenulatum, B. pseudocatenulatum, B. adolescentis, B. animalis, B. gallicum, B. lactis* and *B. bifidum,* more preferably at least one *Bifidobacterium* selected from the group consisting of *B. breve, B. infantis, B. bifidum, B. catenulatum, B. longum*, even more preferably at least one *Bifidobacterium* selected from the group consisting of *B. breve* and *B. longum*, most preferably *B. breve*. Preferably the composition comprises at least two different *Bifidobacterium* species, subspecies or strains. The present composition preferably comprises at least one, more preferably at least two, even more preferably at least three, most preferably at least four different *Bifidobacterium* species. The present composition preferably comprises at least one, more preferably at least two, even more preferably at least three, most preferably at least four different *Bifidobacterium* strains. The above-mentioned combinations commonly aim to increase the diversity and/or the quantity of microorganisms in the intestine of the caesarean section delivered infant. This beneficially affects the infant, providing various health benefits.

Preferably the present composition contains a *Lactobacillus* selected from the group consisting of *L. casei, L. reuteri, L paracasei, L. rhamnosus, L. acidophilus, L. johnsonii, L. lactis, L. salivarius, L. crispatus, L. gasseri, L. zeae, L. fermentum* and *L*. *plantarum*, more preferably *L. casei, L. paracasei, L. rhamnosus, L. johnsonii, L. acidophilus, L. fermentum* and most preferably *L. paracasei*. Even more preferably the present composition comprises *Bifidobacterium breve* and/or *Lactobacillus paracasei*, because the growth of these bacteria in impaired in the intestine of cesarean delivered infants. The further increased biodiversity will have a stimulatory effect on health of the newborn delivered by caesarean section.

### Formulae

The present composition is preferably enterally administered, more preferably orally. The present composition is preferably an infant formula. The present composition is preferably a synthetic formula, prepared by admixing different ingredients. The present composition is not a naturally (non-treated) occurring mammalian milk, e.g. not human breast milk. The present composition can be advantageously used as a complete nutrition for infants. The present composition preferably comprises lipid, protein and carbohydrate and is preferably administered as a liquid food. The term "liquid food" as used in the present invention includes dry food (e.g. powders) which are accompanied with instructions so as to admix said dry food mixture with a suitable liquid (e.g. water).

The present composition preferably provides nutrition to the infant, and comprises a lipid component, a protein component and a carbohydrate component. The lipid component preferably provides 5 to 50% of the total calories, the protein component preferably provides 5 to 50% of the total calories, and the carbohydrate component preferably provides 15 to 90% of the total calories. The present composition is preferably used as an infant formula, wherein the lipid component provides 35 to 50% of the total calories, the protein component provides 7.5 to 12.5% of the total calories, and the carbohydrate component provides 40 to 55% of the total calories. For calculation of the % of total calories for the protein component, the total of energy provided by the proteins, peptides and amino acids needs to be taken.

Preferably the lipid component comprises a combination of at least one lipid selected from the group consisting of vegetable lipids and animal lipids and at least one oil selected from the group consisting of fish, animal, vegetable, algae, fungal and bacterial oil. Preferably, the lipid comprises at least 12 mg/100 kcal α-linolenic acid (ALA). Preferably the lipid composition has a wt/wt ratio of linoleic acid (LA) and ALA between 5 and 15, more preferably 5 to 10. Preferably the amount of trans fatty acids is below 4 wt.% based on total lipid. A high content of trans fatty acids compromises the intestinal barrier.

The protein component used in the present composition preferably comprises at least one selected from the group consisting of non-human animal proteins (such as milk proteins, meat proteins and egg proteins), vegetable proteins (such as soy protein, wheat protein, rice protein, potato protein and pea protein), free amino acids and mixtures thereof. Cow's milk derived nitrogen source, particularly cow milk protein proteins such as casein and whey proteins are particularly preferred. Preferably the protein component comprises intact proteins, more preferably intact bovine whey proteins and/or intact bovine casein proteins. As the present composition is suitably used to reduce the allergic reaction in an infant, the protein of the infant formula is preferably selected from the group consisting of hydrolyzed milk protein (e.g. hydrolyzed casein and/or hydrolyzed whey protein), vegetable protein and/or amino acids. The use of these hydrolyzed proteins further reduces the allergic reactions of the infant. The use of these hydrolyzed proteins advantageously improves the absorption of the dietary protein component by the immature intestine of caesarean delivered infants.

Preferably the composition comprises digestible carbohydrates. The digestible carbohydrates used in the present composition are preferably selected from the group consisting of sucrose, lactose, glucose, fructose, corn syrup solids, starch and maltodextrins, and mixtures thereof, more preferably lactose.

Stool irregularities (e.g. hard stools, insufficient stool volume, diarrhea) are a major problem in many babies, including especially caesarean section delivered infants. It was found that stool problems may be reduced by administering the present LC-PUFA in liquid food. The present composition preferably has an osmolality between 50 and 500 mOsm/kg, more preferably between 100 and 400 mOsm/kg. In view of the above, it is also important that the liquid food does not have an excessive caloric density, however still provides sufficient calories to feed the subject. Hence, the liquid food preferably has a caloric density between 0.1 and 2.5 kcal/ml, even more preferably a caloric density of between 0.5 and 1.5 kcal/ml, most preferably between 0.6 and 0.8 kcal/ml.

### Application

The present invention provides a composition for use as defined in claim 1, said method comprising administering the present composition comprising LC-PUFA.

. The disorders are preferably selected from the group consisting of intestinal disorders caused by a flora low in bifidobacteria content. Preferably the disorder is selected from the group consisting of infection and atopic disease. The present composition is preferably administered to the infant delivered via caesarean section in the first year of life, preferably within 3 months after birth, even more preferably within two weeks after birth, even more preferably within 100 hours, more preferably within 72 hours, most preferably within 48 hours after birth.

The present composition for use as defined in claim 1 preferably comprises the steps a) admixing (i) a nutritionally or pharmaceutically acceptable liquid; and (ii) a dry composition, wherein the dry composition (ii) comprises LC-PUFA, preferably LC-PUFA and nucleotides; and step b) administering the composition obtained in step a) to an infant born via caesarean section.

Administration of a composition comprising LC-PUFA results in a decreased translocation of (nosocomial) pathogenic micro-organisms, such as *Eschericia coli*, other Gram-negative species belonging to the genera *Aeromonas, Klebsiella, Enterobacter, Pseudomonas, Proteus*, and *Acinetobacter* and Gram-positive species such as *Enterococcus, Staphylococcus, Streptococcus, Bacillus,* and *Clostridium*, viruses and/or fungi, preferably *Escherichia coli* (*E. coli*). Hence, the present composition is preferably used in a method for treatment and/or prevention of infection and/or diarrhea in infants delivered by caesarean section, said method comprising administering the present composition to an infant delivered by caesarean section. In a preferred embodiment the present composition is used for the treatment and/or prevention of infection caused by *Eschericia coli, Aeromonas, Klebsiella*, *Enterobacter and*/*or Pseudomonas*, more preferably *E. coli.*

Administration of the present composition comprising LC-PUFA results in a decreased translocation of allergens across the intestinal barrier. Hence, the present composition is preferably used in a method for treatment or prevention of allergy, preferably food allergy, atopic eczema (e.g. atopic dermatitis), asthma, allergic rhinitis and/or allergic conjunctivitis in infants delivered by caesarean section, said method comprising administering said composition to an infant delivered by caesarean section. Preferably the present composition is used for the treatment and/or prevention of atopic dermatitis.

Furthermore, administration of the present composition strengthens the immune system. Hence, the present composition comprising LC-PUFA is advantageously used in a method for treatment and/or prevention of systemic infections and/or inflammation in infants delivered by caesarean section. In a preferred method, the present composition is used for the treatment and/or prevention of, necrotizing enterocolitis.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Molecular characterization of intestinal microbiota in infants born by vaginal delivery vs. caesarean delivery

In the present study the influence of mode of delivery (caesarean delivery versus vaginal delivery) on the intestinal microbial composition at the third day of life was studied using by PCR amplification with species-specific primers for ten *Bifidobacterium* species, three *Ruminococcus* species and one *Bacteroides* species.

The microbial DNA was extracted and analysed according to Favier et al, Environ Microbiol 2002; 68:219-226 and Satokari et al, Appl Environ Microbiol 2001; 67:504-513; Satorkari et al System Appl Microbiol 2003; 26:572-584.

The results of the *Bifidobacterium* and other species detected in faecal samples of 21 newborns after caesarean delivery obtained at the 3rd day of life are given in Table 1. Table 2 gives the *Bifidobacterium* and other species detected in faecal samples of 21 newborns after vaginal delivery obtained at the 3rd day of life. No signal specific for the species *B. dentium, B. angulatum, B. lactis, Ruminococcus bromii, Ruminococcus callidus* and *Ruminococcus obeum* was observed in the faeces of infants delivered by caesarean section as well as in the faeces of vaginally delivered infants.

**Table 1: Caesarean section delivered infants**

| NEWBORN | *B. breve* | *B. infantis* | *B. bifidum* | *B. catenulatum group* | *B. adolescentis* | *B. longum* | *B. gallicum* | *Bacteroides fragilis* |
|---|---|---|---|---|---|---|---|---|
| 1 | - | - | - | - | - | - | - | - |
| 2 | - | - | - | - | - | ++ | - | - |
| 3 | - | - | - | - | - | - | - | - |
| 4 | - | - | - | - | - | - | - | - |
| 5 | - | - | - | - | - | - | - | - |
| 6 | - | - | - | - | - | - | - | - |
| 7 | - | - | - | - | - | - | - | - |
| 8 | - | - | - | - | - | - | - | - |
| 9 | - | - | - | - | - | - | - | - |
| 10 | - | - | - | - | - | - | ++ | - |
| 11 | - | - | - | - | - | - | - | - |
| 12 | - | - | - | - | - | - | - | - |
| 13 | - | - | - | - | - | - | - | - |
| 16 | - | - | - | - | - | - | - | - |
| 17 | - | - | - | - | - | - | - | - |
| 18 | - | - | - | - | - | - | - | - |
| 19 | - | - | - | - | - | - | - | - |
| 20 | - | - | - | - | - | - | - | - |
| 21 | - | - | - | - | - | - | - | - |
| 22 | - | - | - | - | - | - | - | - |
| 23 | - | - | - | - | - | - | - | - |

| | | | | | | | | |
|---|---|---|---|---|---|---|---|---|
| ( - ) = no amplification; (+/-) = weak amplification; ( + ) = positive amplification; ( ++ ) **=** strong amplification | | | | | | | | |

It can be concluded that the microbial flora of an infant born via caesarean section differs from that of an infant born via the vaginal route. Not only is the amount of bifidobacteria and other species quantitatively much lower, also on a species level the flora of caesarean section delivered infants is less diverse. Since *Bifidobacterium* is the dominant genus in infant's flora these results are generalised to less intestinal bacteria and a less diverse intestinal flora in caesarean section delivered infants, leaving the intestine more susceptible to colonisation by (nocosomial) pathogens.

These results are indicative for the advantageous use of the composition and method according to the present invention, e.g. a method for feeding babies born via caesarean section in order to decrease effects of intestinal pathogens and stimulate a healthy intestinal flora and consequently prevent infection, stimulate a healthy immune system and stimulate intestinal maturation, in particular in infants delivered by caesarean section.

**Table 2: Vaginally delivered infants**

| NEWBORN | *B. breve* | *B. infantis* | *B. bifidum* | *B. catenulatum group* | *B. adolescentis* | *B. longum* | *B. gallicum* | *Bacteroides fragilis* |
|---|---|---|---|---|---|---|---|---|
| 1a | - | + | - | - | - | ++ | - | - |
| 2a | +/- | - | ++ | ++ | - | ++ | - | - |
| 3a | - | - | - | + | - | - | - | - |
| 4a | +/- | - | - | ++ | + | + | - | - |
| 5a | +/- | - | ++ | ++ | - | ++ | ++ | - |
| 6a | - | - | +/- | ++ | - | ++ | - | - |
| 7a | - | - | +/- | ++ | ++ | - | - | - |
| 8a | ++ | ++ | - | + | ++ | - | - | - |
| 9a | - | - | - | + | ++ | + | - | - |
| 10a | ++ | - | - | + | + | - | - | - |
| 11a | ++ | - | ++ | ++ | - | ++ | - | + |
| 12a | + | + | + | + | - | ++ | - | + |
| 13a | +/- | - | - | + | - | + | - | - |
| 16a | - | - | - | ++ | - | + | - | - |
| 17a | +/- | - | + | + | - | + | - | - |
| 18a | +/- | - | + | + | - | + | - | - |
| 19a | + | - | - | + | - | + | - | - |
| 20a | - | - | - | + | - | + | - | - |
| 21a | - | - | - | + | ++ | + | - | - |
| 22a | - | + | - | ++ | - | + | - | - |
| 23a | + | - | ++ | ++ | - | + | - | - |

| | | | | | | | | |
|---|---|---|---|---|---|---|---|---|
| ( - ) = no amplification; (+/-) = weak amplification; ( + ) = positive amplification; (++) = strong amplification | | | | | | | | |

### Example 2: Effect of LC-PUFA on barrier integrity

5 Monolayers (MC) of intestinal epithelial cell lines T84 were incubated in the luminal compartment with different 100 µM polyunsaturated fatty acids ARA (arachidonic acid; 5,8,11,14-eicosatetraenoic acid), DHA (cis-4,7,10,13,16,19 docosahexaenoic acid), EPA (eicosapentaenoic acid) or control palmitic (C 16:0) acid (Palm) (Sigma, St. Louis, USA) for 0, 24, 48 and 72 hr. The epithelial barrier function was determined by measuring the transepithelial resistance (TER, Ω.cm²) by epithelial volt-ohm meter (EVOM; World Precision Instruments, Germany) and permeability for 4kD FITC dextran (paracellular permeability marker, Sigma, USA).

Results of the effect of fatty acids (100 µM) on spontaneous barrier integrity after 72 hr incubation are given in Table 3. Table 3 shows that the LC-PUFA's ARA, EPA and DHA reduce the molecular flux and improve epithelial resistance. In contrast the control palmitic acid has the opposite effects, i.e. compromises barrier integrity. These results are indicative for the advantageous use of EPA, DHA and ARA, and in particularly ARA in the composition according to the present invention and for use in a method according to the present invention, e.g. in a method for improving barrier integrity in infants delivered by caesarean section. Also gamma-linolenic acid (GLA) showed a positive effect on basal barrier integrity resistance and flux.

**Table 3**

| Ingredient (LC-PUFA) | Flux | Resistance (TER) |
|---|---|---|
| Control | 79 | 1090 |
| Palmitic acid | 161 | 831 |
| DHA | 72 | 1574 |
| ARA | 28 | 1816 |
| EPA | 65 | 1493 |

### Example 3: Effect of LC-PUFA on IL-4 mediated barrier disruption

Monolayers (MC) of intestinal epithelial cell lines T84 were incubated in the presence of IL-4 (2 ng/ml, serosal compartment, Sigma, USA ) with or without polyunsaturated fatty acids ARA, DHA, GLA, EPA, or control palmitic acid (10 µM or 100 µM, mucosal compartment, Sigma, St. Louis, USA). Cells were pre-incubated with ARA, DHA, EPA, or palmitic acid for 48 hr prior to the IL-4 incubation. The co-incubation of PUFA's and palmitic acid with IL-4 was continued for another 48 hr, while culture medium and additives were changed every 24 hr. The epithelial barrier function was determined by measuring the transepithelial resistance (TER) and permeability as described in example 2.

Results of the effect of ARA, DHA, EPA and palmitic acid (100 µM) on IL-4 mediated barrier disruption are given in Table 4. Table 4 shows that the LC-PUFA's ARA, DHA and EPA inhibit the increased flux caused by IL-4. In contrast palmitic acid had a detrimental effect and decreased barrier disruption compared to control. These results are indicative for the advantageous use of ARA, DHA, and EPA in clinical and infant nutrition formulations to prevent or reduce IL-4 mediated barrier disruption, e.g. as occurs in food or cows milk allergy. These results are indicative for the advantageous use of EPA, DHA and ARA, and in particularly ARA in the composition according to the present invention and for use in a method according to the present invention, particularly in a method for treatment and/or prevention of allergy, atopic eczema, asthma, allergic rhinitis, and/or allergic conjunctivitis in infants delivered by caesarean section.

**Table 4**

| Ingredient (LC-PUFA) | IL-4 Flux | IL-4 TER |
|---|---|---|
| Control | 582 | 374 |
| Palmitic acid | 777 | 321 |
| DHA | 271 | 547 |
| ARA | 218 | 636 |
| EPA | 228 | 539 |

### Example 4: Flora of caesarean section delivered infants.

The bifidobacterial content in the feces of infants delivered via caesarean section was determined. The percentage of the genus *Bifidobacterium* as a total of total bacteria in the first week was 4.3% in caesarean section delivered infants (n=44) versus 19.8% in vaginally delivered infants (n=28). The percentage *E. coli* was still 11.8% after 6 weeks when the infants were fed an normal diet. (see Table 5).

**Table 5:**

| Infants | *Bifidobacteria* first week (%) | *E. coli* Week 6 (%) |
|---|---|---|
| Vaginal delivery | 19.8 | |
| C-section | 4.3 | 11.8 |

These results indicate the desirability of the administration of the present LC-PUFA's to infants born via caesarean section.

### Example 6: Method for feeding babies born via caesarean section

Within two days after the infant is born via caesarean section, a nutritional composition is administered which contains (per liter):
energy 672 Kcal; Protein 14 g; Whey: Casein ratio 60:40; Fat 36 g; Carbohydrate 73 g; Vitamin A 500 µg RE; Mixed natural carotenoids 35 µg RE; Vitamin D3 12 µg; Vitamin E 12 mg; Vitamin K1 45.0 µg; Vitamin B.sub.1 (thiamin) 400 µg; Vitamin B.sub.2 (riboflavin) 1000 µg; Vitamin B.sub.6 (pyridoxine) 400 vg; Vitamin B.sub.12 (cyanacobalmine) 2.0 µg; Niacin 4.1 mg; Folic Acid 120 µg; Pantothenic Acid 2800 mcg; Biotin 18 µg; Vitamin C (ascorbic acid) 86 mg; Choline 100 mg; Inositol 33 mg; Taurine 67 mg, Calcium 510 mg; Phosphorous 290 mg; Magnesium 50 mg; Iron 7.0 mg ; Zinc 4.9 mg; Manganese 84 µg; Copper 330 µg; Iodine 100 µg; Sodium 170 mg; Potassium 740 mg; Chloride 460 mg and Selenium 14 µg; wherein the fat content includes 0.65 wt.% LC-PUFA based on total fatty acids, including 0.35 wt.% ARA, 0.05 wt.% EPA, and 0.2 wt.% DHA further comprising 3.6 g transgalacto-oligosaccharides obtained from Elix'or^{™} (Borculo Domo Ingredients, Netherlands) and 0.4 g fructo-oligosaccharides obtained from RaftilineHP^{™} (Orafti Active Food Ingredients, Belgium) and about 20 mg nucleotides (about 6.4 mg CMP, about 4 mg UMP, about 6 mg AMP, about 1.4 mg GMP and about 2.4 mg IMP).

## Claims

1. Composition for use in a method for decreasing translocation of pathogenic microorganisms or allergens across the intestinal barrier and/or for treatment or prevention of infection, diarrhea, intestinal inflammation, allergy, atopic eczema, asthma, allergic rhinitis and/or allergic conjuctivitis in an infant born via caesarean section, wherein said method comprises administering said composition to an infant delivered by caesarean section, wherein said composition comprises long chain polyunsaturated fatty acid (LC-PUFA), said composition not being human breast milk.

2. Composition for use in a method according to claim 1, wherein the method comprises
(i) admixing I) a nutritionally or pharmaceutically acceptable liquid; and II) a dry composition comprising long chain polyunsaturated fatty acid (LC-PUFA);
(ii) administering the composition obtained in step (i) to the infant.

3. Composition for use in a method according to claim 1 or 2, wherein the LC-PUFA is selected from the group consisting of eicosapentaenoic acid, docosahexaenoic acid and arachidonic acid.

4. Composition for use in a method according to any of claims 2 or 3, wherein the composition obtained in step (i) comprises 0.1 to 15 wt.% LC-PUFA based on weight of total fat in the composition.

5. Composition for use in a method according to any of claims 2-4, wherein the composition obtained in step (i) comprises less than 3 wt.% LC-PUFA based on weight of total fat present in the composition;
a. the omega-3 LC-PUFA is below 1 wt.% of the total fat content;
b. the omega-6 LC-PUFA is below 2 wt.% of the total fat content; and
c. the arachidonic acid content is below 1 wt.% of the total fat content.

6. Composition for use in a method according to any of the preceding claims, being an infant formula, further comprising at least one selected from the group consisting of (a) nucleotide and (b) nucleotide precursor selected from the group consisting of nucleosides, purine bases, pyridine bases, ribose and deoxyribose.

7. Composition for use in a method according to claim 6,scomprising at least one nucleotide selected from the group consisting of cytidine 5'-monophosphate, uridine 5'-monophosphate, adenosine 5'-monophosphate, guanosine 5'-monophosphate, inosine 5'-monophosphate, and sodium salts of one of the before mentioned nucleotides.

8. Composition for use in a method according to claim 6 or 7, wherein the composition comprises 5 to 100 mg nucleotides per 100 gram dry weight of the composition.

9. Composition for use in a method according to any of claims 2-8, wherein the composition obtained in step (i) further comprises at least one non-digestible oligosaccharide selected from the group consisting of fructans (including inulin), fructo-oligosaccharides, non-digestible dextrins, galacto-oligosaccharides (including transgalacto-oligosaccharides), xylo-oligosaccharides, arabino-oligosaccharides, arabino-galacto-oligosaccharides, gluco-oligosaccharides (including cyclodextrins and gentio-oligosaccharides), chito-oligosaccharides, gluco-manno-oligosaccharides, galacto-manno-oligosaccharides, mannan-oligosaccharides, fuco-oligosaccharides, galacturonic oligosaccharides, guluronic acid oligosaccharides, mannuronic acid oligosaccharides, iduronic acid oligosaccharides, riburonic acid oligosaccharides, glucuronic acid oligosaccharides and mixtures thereof.

10. Composition for use in a method according to any of claims 2-9, wherein the composition obtained in step (i) comprises trans-galacto-oligosaccharides and/or fructooligosaccharides.

11. Composition for use in a method according to any of the claims 2-10, wherein the composition obtained in step (i) comprises galacturonic acid oligosaccharides.

12. Composition for use in a method according to any of the claims 2-11, wherein the composition obtained in step (i) further comprises at least one bacterial species belonging to the genus Bifidobacterium or Lactobacillus.

13. Composition for use in a method according to any one of claims 2-12 wherein the composition obtained in step (i) further comprises lipid, protein and carbohydrate and wherein the lipid component provides 5 to 50% of the total calories, the protein component provides 5 to 50% of the total calories, and the carbohydrate component provides 15 to 90% of the total calories.

## Patentansprüche

1. Stoffgemisch zur Verwendung in einem Verfahren zum Verringern einer Translokation von pathogenen Mikroorganismen oder Allergenen durch die Darmbarriere und/oder für Behandlung oder Prävention von Infektion, Durchfall, Darmentzündung, Allergie, atopischem Ekzem, Asthma, allergischer Rhinitis und/oder allergischer Konjunktivitis in einem durch Kaiserschnitt geborenen Säugling, wobei das Verfahren ein Verabreichen des Stoffgemisches an ein durch Kaiserschnitt entbundenen Säugling aufweist, wobei das Stoffgemisch langkettige, mehrfach ungesättigte Fettsäuren (LC-PUFA) aufweist und das Stoffgemisch nicht menschliche Muttermilch ist.

2. Stoffgemisch zur Verwendung in einem Verfahren nach Anspruch 1, wobei das Verfahren aufweist
(i) Beimengen I) einer alimentär oder pharmazeutisch verträglichen Flüssigkeit; und II) eines trockenen Stoffgemisches, das langkettige mehrfach ungesättigte Fettsäuren (LC-PUFA) aufweist;
(ii) Verabreichen des in Schritt (i) erhaltenen Stoffgemisches an den Säugling.

3. Stoffgemisch zur Verwendung in einem Verfahren nach Anspruch 1 oder 2, wobei die LC-PUFA aus der Gruppe ausgewählt wird, die aus Eicosapentaensäure, Docosahexaensäure und Arachidonsäure besteht.

4. Stoffgemisch zur Verwendung in einem Verfahren nach Anspruch 2 oder 3, wobei das in Schritt (i) erhaltene Stoffgemisch 0.1 bis 15 % Massenanteil LC-PUFA aufweist, basierend auf einem Gewicht des gesamten Fettes in dem Stoffgemisch.

5. Stoffgemisch zur Verwendung in einem Verfahren nach einem der Ansprüche 2-4, wobei das in Schritt (i) erhaltene Stoffgemisch weniger als 3 % Massenanteil LC-PUFA aufweist, basierend auf einem Gewicht des gesamten Fettes, das in dem Stoffgemisch vorhanden ist;
a. die Omega-3 LC-PUFA ist unter 1 % Massenanteil des gesamten Fettgehalts;
b. die Omega-6 LC-PUFA ist unter 2 % Massenanteil des gesamten Fettgehalts; und
c. der Arachidonsäuregehalt ist unter 1 % Massenanteil des gesamten Fettgehalts.

6. Stoffgemisch zur Verwendung in einem Verfahren nach einem der vorigen Ansprüche, wobei das Stoffgemisch Säuglingsnahrung ist und des Weiteren mindestens einen Stoff ausgewählt aus der Gruppe bestehend aus (a) Nukleotid und (b) Vorläufernukleotid aufweist, wobei das Vorläufernukleotid ausgewählt ist aus der Gruppe bestehend aus Nukleosiden, Purinbasen, Pyridinbasen, Ribose und Desoxyribose.

7. Stoffgemisch zur Verwendung in einem Verfahren nach Anspruch 6, das mindestens ein Nukleotid ausgewählt aus der Gruppe aufweist, die aus Cytidin 5'-Monophosphat, Uridin 5'-Monophosphat, Adenosin 5'-Monophosphat, Guanosin 5'-Monophosphat, Inosin 5'-Monophosphat und Natriumsalzen eines der vorher erwähnten Nukleotiden besteht.

8. Stoffgemisch zur Verwendung in einem Verfahren nach Anspruch 6 oder 7, wobei das Stoffgemisch 5 bis 100 mg Nukleotide auf 100 Gramm Trockengewicht des Stoffgemisches aufweist.

9. Stoffgemisch zur Verwendung in einem Verfahren nach einem der Ansprüche 2-8, wobei das in Schritt (i) erhaltene Stoffgemisch weiter mindestens ein unverdauliches Oligosaccharid aufweist, ausgewählt aus der Gruppe bestehend aus Fruktanen (Inulin umfassend), Fructo-Oligosacchariden, unverdaulichen Dextrinen, Galacto-Oligosacchariden (Transgalacto-Oligosacchariden umfassend), Xylo-Oligosacchariden, Arabino-Oligosacchariden, Arabino-Galacto-Oligosacchariden, Gluco-Oligosacchariden (Cyclodextrinen und Genito-Oligosacchariden umfassend), Chito-Oligosacchariden, Gluco-Manno-Oligosacchariden, Galacto-Manno-Oligosacchariden, Mannan-Oligosacchariden, Fuco-Oligosacchariden, Galacturon-Oligosacchariden, Guluronsäure-Oligosacchariden, Mannuronsäure-Oligosacchariden, Iduronsäure-Oligosacchariden, Ribonsäure-Oligosacchariden, Glucuronsäure-Oligosacchariden und Mischungen daraus.

10. Stoffgemisch zur Verwendung in einem Verfahren nach einem der Ansprüche 2-9, wobei das in Schritt (i) erhaltene Stoffgemisch Trans-Galacto-Oligosaccharide und/oder Fructooligosaccharide aufweist.

11. Stoffgemisch zur Verwendung in einem Verfahren nach einem der Ansprüche 2-10, wobei das in Schritt (i) erhaltene Stoffgemisch Galacturonsäure-Oligosaccharide aufweist.

12. Stoffgemisch zur Verwendung in einem Verfahren nach einem der Ansprüche 2-11, wobei das in Schritt (i) erhaltene Stoffgemisch mindestens eine Bakterienspezies, die zu der Gattung Bifidobakterien oder Laktobazillen gehört, aufweist.

13. Stoffgemisch zur Verwendung in einem Verfahren nach einem der Ansprüche 2-12, wobei das in Schritt (i) erhaltene Stoffgemisch weiter Lipid, Protein und Kohlenhydrat aufweist und wobei die Lipidkomponente 5 bis 50 % der Gesamtkalorien bereitstellt, die Proteinkomponente 5 bis 50 % der Gesamtkalorien bereitstellt und die Kohlenhydratkomponente 15 bis 90 % der Gesamtkalorien bereitstellt.

## Revendications

1. Composition pour son utilisation dans un procédé de réduction de la translocation de microorganismes pathogènes ou d'allergènes à travers la barrière intestinale et/ou pour le traitement ou la prévention des infections, des diarrhées, des inflammations intestinales, des allergies, de l'eczéma atopique, de l'asthme, de la rhinite allergique et/ou de la conjonctivite allergique chez un nouveau-né né par césarienne, dans laquelle ladite procédé comprend l'administration de ladite composition à un nouveau-né né par césarienne, dans laquelle ladite composition comprend un acide gras polyinsaturé à chaîne longue (LC-PUFA), ladite composition n'étant pas du lait maternel humain.

2. Composition pour son utilisation dans un procédé selon la revendication 1, dans laquelle le procédé comprend
(i) le mélange I) d'un liquide acceptable sur le plan nutritionnel ou pharmaceutique ; et II) d'une composition sèche comprenant un acide gras polyinsaturé à longue chaîne (LC-PUFA) ;
(ii) l'administration de la composition obtenue dans l'étape (i) au nouveau-né.

3. Composition pour son utilisation dans un procédé selon la revendication 1 ou 2, dans laquelle le LC-PUFA est sélectionné dans le groupe constitué de l'acide éicosapentaénoïque, de l'acide docosahexaénoïque et de l'acide arachidonique.

4. Composition pour son utilisation dans un procédé selon l'une quelconque des revendications 2 ou 3, dans laquelle la composition obtenue dans l'étape (i) comprend de 0,1 à 15 % en poids de LC-PUFA sur la base du poids des matières grasses totales de la composition.

5. Composition pour son utilisation dans un procédé selon l'une quelconque des revendications 2 à 4, dans laquelle la composition obtenue dans l'étape (i) comprend moins de 3 % en poids de LC-PUFA sur la base du poids des matières grasses totales présentes dans la composition ;
a. la teneur en oméga-3 LC-PUFA est inférieure à 1 % en poids de la teneur totale matières grasses ;
b. la teneur en oméga-6 LC-PUFA est inférieure à 2 % en poids de la teneur totale en matières grasses ; et
c. la teneur en acide arachidonique est inférieure à 1 % en poids de la teneur totale en matières grasses.

6. Composition pour son utilisation dans un procédé selon l'une quelconque des revendications précédentes, étant un lait artificiel pour nouveau-né, comprenant en outre au moins un élément sélectionné dans le groupe consistant en (a) des nucléotides et (b) des précurseurs de nucléotides sélectionnés dans le groupe consistant en des nucléosides, des bases puriques, des bases pyrimidiques, le ribose et le désoxyribose.

7. Composition pour son utilisation dans un procédé selon la revendication 6, comprenant au moins un nucléotide sélectionné dans le groupe consistant en la cytidine 5'-monophosphate, l'uridine 5'-monophosphate, l'adénosine 5'-monophosphate, la guanosine 5'-monophosphate, l'inosine 5'-monophosphate, et les sels de sodium de l'un des nucléotides susmentionnés.

8. Composition pour son utilisation dans un procédé selon l'une quelconque des revendications 6 ou 7, dans laquelle la composition comprend de 5 à 100 mg de nucléotides pour 100 grammes de poids sec de la composition.

9. Composition pour son utilisation dans un procédé selon l'une quelconque des revendications 2 à 8, dans laquelle la composition obtenue dans l'étape (i) comprend en outre au moins un oligosaccharide non digestible sélectionné dans le groupe consistant en les fructanes (y compris l'inuline), les fructo-oligosaccharides, les dextrines non digestibles, les galacto-oligosaccharides (y compris les transgalacto-oligosaccharides), les xylo-oligosaccharides, les arabino-oligosaccharides, les arabino-galacto-oligosaccharides, les gluco-oligosaccharides (y compris les cyclodextrines et les gentio-oligosaccharides), les chito-oligosaccharides, les gluco-manno-oligosaccharides, les galacto-manno-oligosaccharides, les mannane-oligosaccarides, les fuco-oligosaccharides, les oligosaccharides galacturoniques, les oligosaccharides d'acide guluronique, les oligosaccharides d'acide mannuronique, les oligosaccharides d'acide iduronique, les oligosaccharides d'acide riburonique, les oligosaccharides d'acide glucuronique et leurs mélanges.

10. Composition pour son utilisation dans un procédé selon l'une quelconque des revendications 2 à 9, dans laquelle la composition obtenue dans l'étape (i) comprend des trans-galacto-oligosaccharides et/ou des fructo-oligosaccharides.

11. Composition pour son utilisation dans un procédé selon l'une quelconque des revendications 2 à 10, dans laquelle la composition obtenue dans l'étape (i) comprend des oligosaccharides d'acide galacturonique.

12. Composition pour son utilisation dans un procédé selon l'une quelconque des revendications 2 à 11, dans laquelle la composition obtenue dans l'étape (i) comprend en outre au moins une espèce bactérienne appartenant au genre *Bifidobacterium* ou *Lactobacillus.*

13. Composition pour son utilisation dans un procédé selon l'une quelconque des revendications 2 à 12, dans laquelle la composition obtenue dans l'étape (i) comprend en outre un lipide, une protéine et un hydrate de carbone et dans laquelle le composant lipidique fournit 5 à 50 % des calories totales, le composant protéique fournit 5 à 50 % des calories totales et le composant glucidique fournit 15 à 90 % des calories totales.
